# EUROPEAN PATENT APPLICATION

(11) **EP 0 681 852 A1**
(43) Date of publication of application: **15.11.1995**
(21) Application number: 95870046.0
(22) Date of filing: 04.05.1995
(51) Int. Cl.: A61N 5/10, G01T 1/29

(54) **Detector for a radiation therapy transport apparatus**

(30) Priority: 13.05.1994 US 242361
(71) Applicant: LOMA LINDA UNIVERSITY MEDICAL CENTER, Loma Linda, California 92354 (US)
(72) Inventor: Coutrakon, George B., Redlands, California 92373 (US); Lesyna, David A., Redlands, California 92373 (US)
(74) Representative: Fiener, Josef

(57) **Abstract**

A beam sensor is used to detect the shape and position of a charged particle beam in a radiation transport system (10). The sensor (18) is comprised of a movable detector assembly (20) which is movable into and out of the beam path. Ionization chambers (48,50,52) are housed within the detector assembly and contain a substance which is ionizable by the charged particle beam. Each chamber is comprised of rigid plates (40,42,44,46) having patterns of conductive material which produce signals in response to the ionization of the ionizable substance by the beam.

## Description

### Field of the Invention

The present invention is related to transport systems for radiation therapy, particularly proton beam therapy, and to a method of detecting the position and shape of a charged particle beam.

### Background of the Invention

Radiation detectors have been utilized in high energy particle physics laboratories to determine the profile or shape of a charged particle beam and the location of the charged particle beam. Such detectors may, for example, comprise ionization chambers which detect the charged particle beam by measuring the amount of charge liberated by the interaction of the charged particle beam with an ionizable substance. The ionization chamber is typically comprised of at least a pair of plates, each plate representing an anode or a cathode. Upon ionization of the ionizable substance, electrons are collected at the anode and positive ions are collected at the cathode.

Detectors having ionization chambers may be located at various intervals along the beam path and at the terminus of radiation transport systems. By monitoring these detectors, information necessary for focussing or adjusting the beam can be obtained. Typically, the particle beam detectors are stationary and remain in the beam path at all times. Accordingly, some beam scattering will occur, thereby causing degradation of the beam. Beam scattering, together with the beam's natural tendency to diverge, adversely affects beam focussing at the target site. While errors in focussing the beam may be acceptable in some applications, medical applications involving radiation therapy, particularly proton beam therapy, require accurate and precise beam characteristics.

In order to alleviate beam degradation, the detectors utilize appropriate materials. In particular, when located along the beam path, the plates of the ionization chambers are comprised of a material which is permeable to the charged particle beam and does not adversely distort or scatter the charged particle beam. Typically, the plates are comprised of a thin layer of gold deposited in a pattern on very thin Mylar™ sheets. The gold layers are connected to signal monitoring devices which collect the signals generated by the charged particles impinging on particular areas of the gold. The signals, derived from the pattern of conductive material, are subsequently processed using algorithms to determine beam profile and location.

In addition to depositing patterns of gold on thin Mylar™ sheets, detectors using ionization chambers have been configured using a plurality of closely spaced wires. Charge is collected on the wires and the characteristics of the particle beam can be determined by measuring the signals generated on the wires. The charged particle beam passes through the ionization chamber and continues along the beam path virtually unaltered.

In contrast to the above discussed ionization chambers spaced along the beam path, ionization chambers located at the terminus of the transport system may be formed of relatively thick plates comprised of conductive and non-conductive materials. These plates, like those located along the beam path, are mounted in a fixed position so as to maintain system alignment. Since these plates are located at the final destination or terminus of the transport system, degradation of the beam due to plate thickness is of less concern, and therefore, the plates can be relatively thick. Beam profile and location are determined using algorithms similar to those used for the previously described thin plates.

There are a number of disadvantages associated with the above mentioned devices. First, the thin sheet devices are difficult to manufacture. Since the beam must travel basically unaltered through the conductive material, the conductive material must be very thin. The manufacturing processes associated with these devices are costly and complex. In addition, due to the thinness of the conductive material, the gold tends to "float" above the Mylar™ sheet, thereby producing imprecise results.

Another disadvantage associated with the thin sheet devices is that the sheets tend to buckle or bow. Because of this, the volumes within the ion chambers can vary. This, in turn, produces inaccurate and un-reproducible results. In addition, in some cases, the bowing may be so severe as to cause arcing between adjacent sheets.

Disadvantages associated with the thick sheet devices include design constraints and complicated and costly manufacturing requirements. Due to their tendency to significantly degrade the beam, thick sheet devices, if used along the beam path, would likely be limited to a pair of non-conductive substrates forming a single ion chamber. In such single ion chambers, all of the beam parameters must be ascertained as a result of the charge collected on a single conductive surface. Complicated patterns of conductive material are thus required to determine beam shape and location. Moreover, in order to adequately collect all of the beam information and transfer the signals generated by the beam impinging on the conductive surface, complicated and costly electronics are required.

Thus, there is a need for a detector which can be used in a charged particle beam transport system of a radiation therapy system to permit control of beam shape and location during transport without significantly degrading the beam. The detector should preferably permit use of multiple ion chambers, and should be easy to manufacture, and configured so as to give accurate, reproducible results.

### Summary of the Invention

The present invention provides a novel beam sensor for detecting the shape of a charged particle beam and the location of the beam relative to the beam path. The preferred embodiment of this invention includes a radiation therapy transport apparatus which guides a beam of charged particles from a charged particle source to a patient at a treatment site. The radiation therapy transport system and associated gantries and treatment sites are best illustrated and described in U.S. Patent No. 4,870,287, issued September 26, 1989 and included herein by reference.

The beam of charged particles propagates within a tube, connected to receive the charged particles, from the source to the treatment site. As the beam propagates within the tube, beam parameters are adjusted by magnets located at various intervals along the tube. A beam sensor is placed in the path of the particle beam for detecting the shape and location of the beam as it travels along the beam path.

The beam sensor, which comprises a detector, is mounted on the tube through which the charged particle beam travels. The detector is movable between a first position and a second position. In the first position, the detector is in the beam path; whereas, in the second position, the detector is outside the beam path. Preferably, the apparatus includes a multitude of such sensors spaced at intervals along the beam path.

The detector of the beam sensor is comprised of at least one ion chamber which contains a substance, such as air, which is ionizable by the charged particle beam. In addition, the ion chamber is comprised of plural rigid plates which include a pattern of conductive material on at least one side of each plate. One of the patterns of conductive material comprises a first set of electrically isolated conductive elongate strips. Another one of the patterns of conductive material comprises a second set of electrically isolated conductive elongate strips. Further, the first set of strips is disposed at an angle relative to the second set of strips. The sets of strips and the other patterns of conductive material produces signals which are generated in response to the ionization of the ionizable substance by the charged particle beam.

In the preferred embodiment, the detector is comprised of three ion chambers. The ionization produced by the passage of the charged particles through an ionizable substance generates a voltage which is measured. The first chamber is bounded by two rigid plates, wherein one of the plates is a quad foil and the other plate is a high voltage plate.

The quad foil of the preferred embodiment is a rigid plate comprised of non-conductive material or substrate and conductive material. The conductive material is divided into four electrically isolated wedge-shaped portions. The wedges are arranged so that their edges are juxtaposed with the edges of an adjacent wedge, such that, when combined, the wedges form a disk.

The high voltage plate of the preferred embodiment, comprised of a substrate and conductive materials, is adapted for connection to a source of high voltage. The high voltage plate has conductive material on opposite sides of the plate and, therefore, is also used to form the second ion chamber. In addition to the high voltage plate, the second ion chamber is comprised of another rigid plate.

The other plate of the second ion chamber is also comprised of conductive material and a substrate. However, the conductive material, located on opposite sides of the plate, is arranged in a pattern of electrically isolated elongate strips. The strips on the first side of the plate are disposed at an angle relative to the strips on the second side of the plate. Thus, this plate, together with yet another rigid plate, comprise the third ion chamber.

The rigid high voltage plate of the third ion chamber is almost identical to the high voltage plate of the first ion chamber. However, the high voltage plate of the third ion chamber has conductive material on only one side of the plate. The side of the plate with conductive material is located within the third ion chamber and faces the opposite direction of the beam path. The high voltage plate of the third ion chamber is connected to the high voltage plate of the first ion chamber and, therefore, is also adapted for connection to a high voltage source.

For ease of description, the term upstream will refer to those elements located toward the source of charged particles and the term downstream will refer to those elements positioned toward the target site. In addition, with respect to the plates, the front of the plate refers to the side of the plate upon which the charged particle beam first impinges and the back of the plate refers to the side of the plate through which the charged particle beam exits.

The plates are arranged such that the quad foil is positioned upstream of the high voltage plate of the first and second ion chambers, which is positioned upstream of the plate located between the second and third ion chambers, which is positioned upstream of the high voltage plate of the third ion chamber relative to the incoming beam of charged particles. The quad foil and the plate located between the second and third ion chambers are connected together so that their signals, in response to the ionization of the ionizable substance, may be simultaneously sent to the signal monitoring device.

### Brief Description of the Drawings

FIGURE 1 is a somewhat schematic perspective view of a radiation therapy transport system connected between a synchrotron and multiple treatment rooms, showing the location of the beam sensors of the present invention.

FIGURE 1A is an enlarged view of one of the beam sensors shown in FIGURE 1.

FIGURE 2 is a cross-sectional view of the beam sensor of FIGURE 1A, taken along the lines 2-2 of FIGURE 1A, illustrating the movable detector assembly.

FIGURE 3 is a partial cross-sectional view, taken along the lines 3-3 of FIGURE 2, illustrating the three ionization chambers of the detector assembly.

FIGURE 4 is an elevational view illustrating the back side of the quad foil of the first ion chamber of the detector assembly of the present invention.

FIGURE 5 is an elevational view illustrating the front side of the high voltage plate of the first ion chamber of the detector assembly.

FIGURE 6 is an elevational view of the back side of the plate of FIGURE 5 illustrating the high voltage plate of the second ion chamber of the detector assembly.

FIGURE 7 is an elevational view illustrating the front side of the plate with the pattern of conductive material forming strips of the second ion chamber of the detector assembly.

FIGURE 8 is an elevational view of the plate of FIGURE 7 illustrating the back side of the plate with the pattern of conductive material forming strips of the third ion chamber of the detector assembly.

FIGURE 9 is an elevational view illustrating the front side of the high voltage plate of the third ion chamber of the detector assembly.

### Detailed Description of the Invention

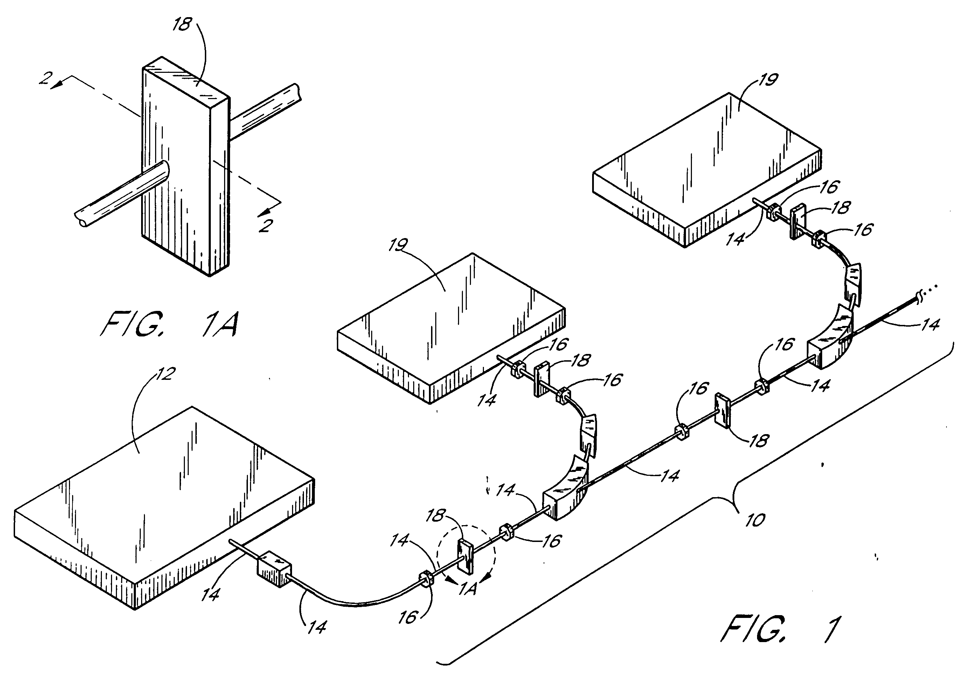

Referring to FIGURE 1, in the preferred embodiment of the invention, a radiation transport system 10 is comprised of an accelerator 12 which emits a beam of charged particles into a beam transport tube 14. A plurality of magnets 16 and beam sensors 18 are located at various intervals along the beam transport tube 14. The beam of charged particles travels along the transport tube 14 until it reaches its final destination or target sites. In the preferred embodiment, the target sites comprise respective treatment rooms 19, each of which contains a modular, isocentric gantry for supporting and rotating a radiation beam transport and delivery system around a patient, as disclosed in U.S. Patents No. 4,917,344 and 5,039,057, which are hereby incorporated herein by reference.

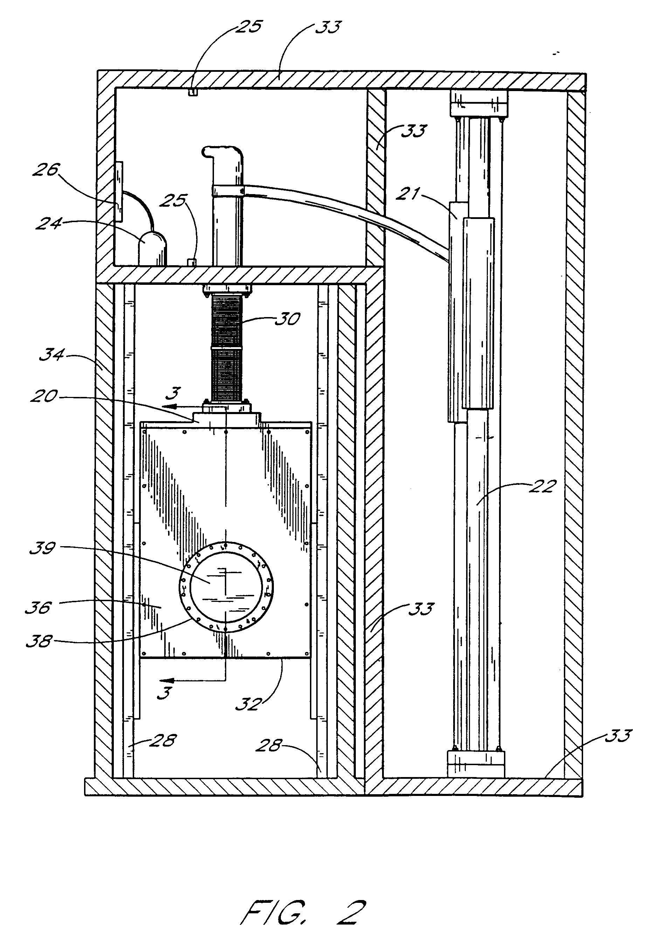

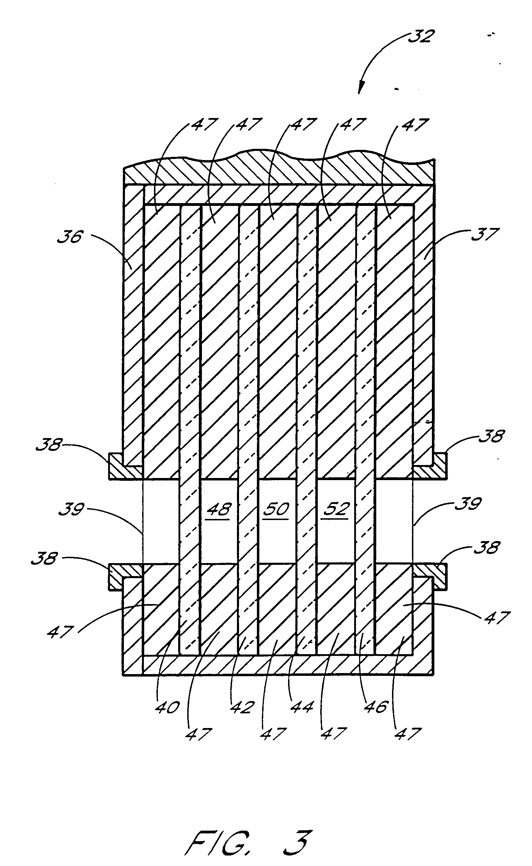

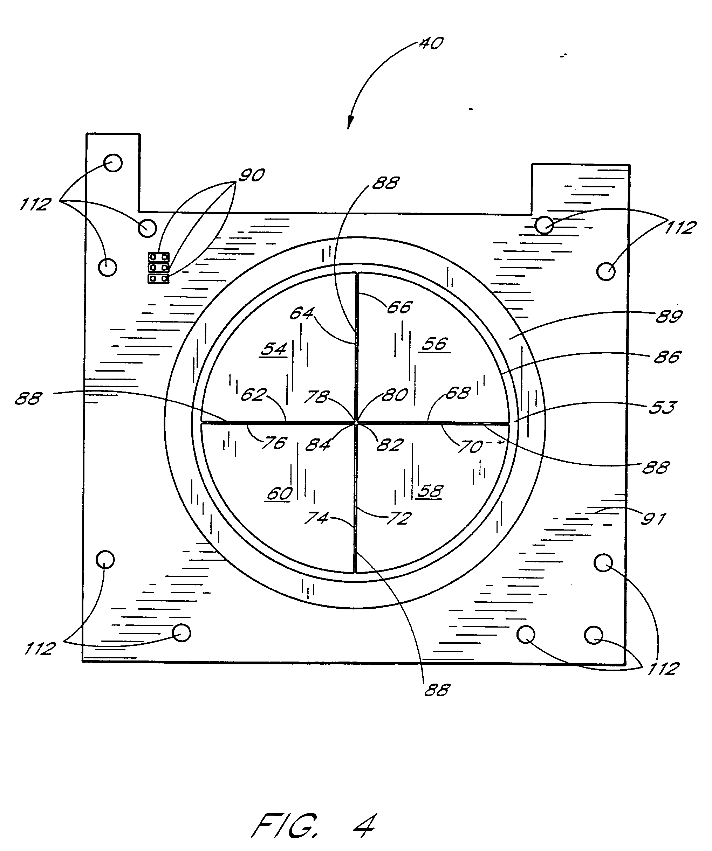

As shown in FIGURES 1A and 2, the beam sensor 18 is comprised of an outer cover 34, a frame 33, a movable detector assembly 20, linear slides 28, a pneumatic actuator 22, an air solenoid 24, switches 25 and an electronic control board 26. Located at the back and front surfaces of the outer cover 34 are ports (not shown). The sensors 18 are connected to the beam transport tube 14, and are in alignment with the beam path, via the ports. Although only a single sensor is depicted in detail in FIGURE 2, it will be understood that all sensors 18 are similar.

The interior of the beam transport tube 14 and the interior of the beam sensor 18 are held under vacuum, so as to reduce the amount of scatter of the charged particle beam. The components are securely sealed together via gaskets, o'rings or other suitable sealing devices in order to maintain zero atmosphere. For example, the beam transport tube 14 seals onto the ports of the outer cover 34 of the sensor 18 and the outer cover 34 of the sensor 18 seals onto the frame 33, thereby creating an air-tight system.

Still referring to FIGURE 2, the movable detector assembly 20, which is attached to the pneumatic actuator 22 via a bracket 21, is comprised of a metallic bellows 30 and an anti-vacuum box 32 which contains an ionizable substance such as air. Of course, since the interior of the beam sensor is under vacuum, the box 32 is tightly sealed to prevent the air from escaping.

The detector assembly 20 is movable between a first position and a second position. In the first position, the anti-vacuum box 32 of the detector assembly 20 is located within the beam path; whereas, in the second position, the anti-vacuum box 32 is outside the beam path. Switches 25 are used to verify that the detector is either in the first position or the second position.

The anti-vacuum box 32 of FIGURE 2 of the detector assembly 20 is comprised of a cover plate 36 and a case 37 (not shown). A circular opening or port 38 is located on the surface of the cover plate 36 and the case 37. Each port 38 is filled with a thin sheet of titanium 39. When the anti-vacuum box 32 is in the first position, the circular ports 38 of the cover plate 36 and the case 37 are in direct alignment with the beam path and with the ports located on the outer cover 34 of the sensor 18.

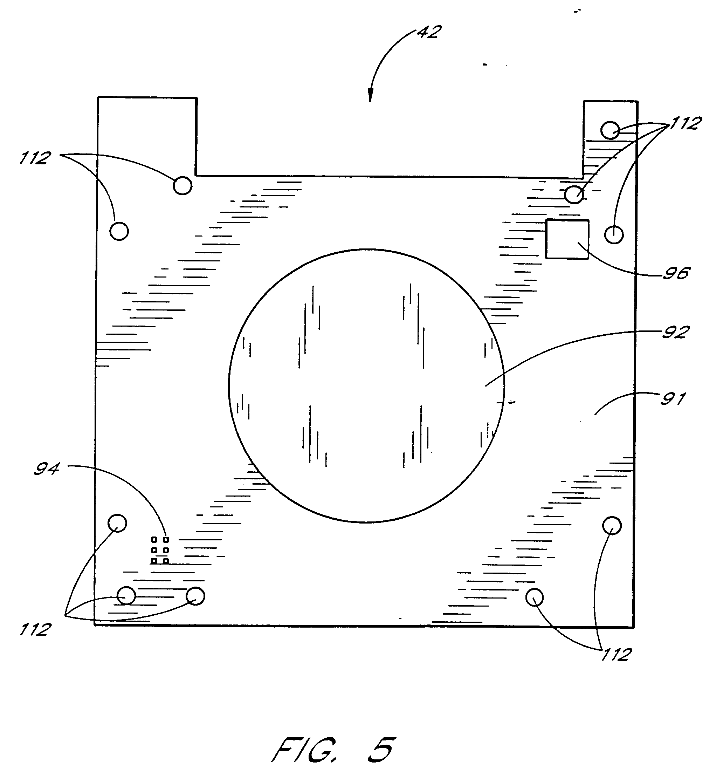

Housed within the anti-vacuum box 32 are four plates 40,42,44,46, as shown in FIGURE 3. Each plate is comprised of a substrate with a pattern of conductive material on one or opposite sides of each plate. In a preferred embodiment, the substrate is G-10 glass-epoxy which is 0.805mm thick. However, combinations of epoxy and fiberglass and other printed circuit board materials may also be used, as is well known by those skilled in the art. The conductive plate material is copper cladding which is approximately 0.04mm thick; however, other conductive materials, such as gold, may also be used.

Still referring to FIGURE 3, spacers 47, made of rigid, non-conductive material or substrate, are located between each pair of plates 40-46 so that each pair of plates 40-46 comprises an ion chamber 48,50,52 of defined volume. Each of the spacers 47 comprises a plate having a through-hole approximately centered therein. The edges of the through-holes define boundaries of the ion chambers, and the holes are aligned so as to allow the charged particle beam to pass through the ion chambers 48, 50, 52. Other configurations of the spacers 47 are also acceptable provided that they do not block or otherwise interfere with the charged particle beam.

The plates 40-46 are sufficiently rigid so that they are not subject to bowing, stretching or other distortions. In a preferred embodiment, the plates 40-46 are 0.020 inch thick with a plate separation (i.e. spacer 47 thickness) of 2.8mm. Such rigidity ensures reproducibility of the defined volume of the chambers 48, 50, 52.

The first ion chamber 48 is comprised of a plate 40 and a plate 42. The plate 40, positioned upstream of the incoming beam of charged particles relative to the plate 42, has a pattern of conductive material on only one side. The conductive material on the plate 40 is located on the back of the plate 40, within the first ion chamber 48. The front of the plate 40 is covered by a layer of non-conductive material or mask with a set of cross-hairs etched within the center. The cross-hairs are used to visually center or locate the plates 40-46 within the anti-vacuum box 32 via the ports 38.

Both sides of the plate 42 are similar in that each has a pattern of conductive material. The conductive material is disk-shaped and is approximately centered on each side.

The second ion chamber 50, as shown in FIGURE 3, is comprised of the plate 42 and a plate 44. The plate 44, similar to the plate 42, also has a pattern of conductive material on opposite sides of the plate 44. The pattern of conductive material on the plate 44, in addition to the other patterns of conductive material on the plates 40,42,46, will be discussed in more detail below.

The plate 44 and a plate 46 make up the third ion chamber 52. The plate 46 has a pattern of conductive material located on the front side of the plate 46, within the third ion chamber 52. The back of the plate 46, however, does not have any conductive material.

The plate 46 is in electronic communication with the plate 42 via a six prong connector, which is connected to a source of high voltage (not shown). Likewise, the plate 40 and plate 44 are adapted for communication via a six prong connector which connects to a signal monitoring device (not shown).

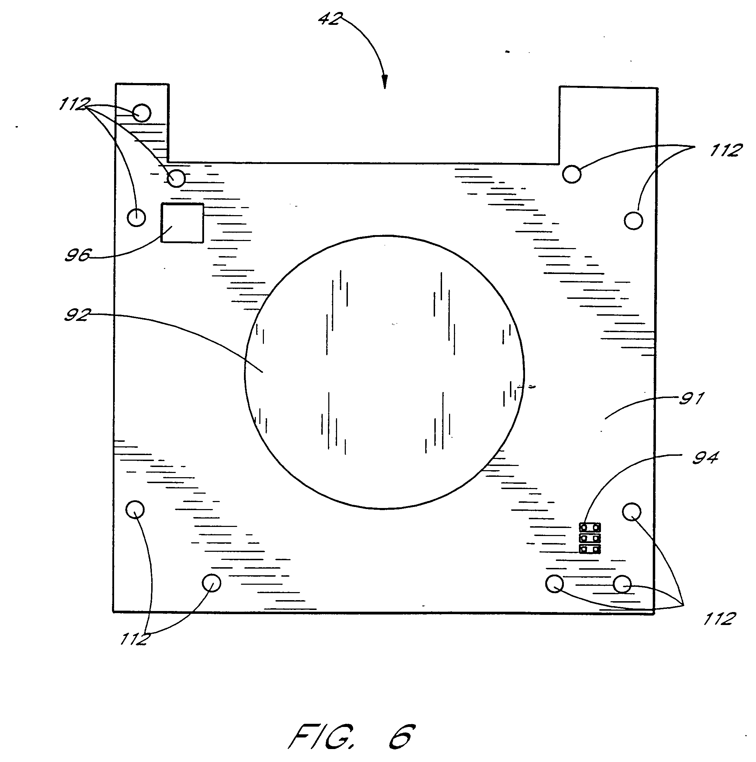

The individual conductive patterns for each of the four plates 40-46 are shown in detail in FIGURES 4-9. FIGURE 4 illustrates the pattern of conductive material on the back side of the plate 40 or quad foil. The conductive material is divided into four electrically isolated wedges 54,56,58,60 which are surrounded by substrate 53,88. The edges 62,64,66,68,70,72,74,76 of each wedge are juxtaposed with an edge of an adjacent wedge. For example, the edges 62,64 of the first wedge 54 are juxtaposed with an edge 66 of the second wedge 56 and an edge 76 of the fourth wedge 60. Each of the wedges 54,56,58,60 has an apex and the apexes 78,80,82,84 of the wedges 54-60 are juxtaposed such that the wedges form sections of a disk 86. The electrically isolated wedges 54-60 are separated from one another by a 0.008 inch thick gap of substrate 88. Each wedge is in electronic communication with a signal monitoring device (not shown) via a six prong connector 90 located on the plate 40.

As shown in FIGURE 4, a thin ring of conductive material 89 surrounds the substrate 53. The ring 89 is a result of the etching process, during the manufacturing of the plate 40, which removes the mask 91. Initially, the entire plate 40 is covered with mask 91.

The patterns of conductive material for opposite sides of the plate 42 are illustrated in FIGURES 5 and 6. The conductive material 92 is disk-shaped and is approximately centered on opposite sides of the plate 42. Each disk of conductive material 92, which is surrounded by mask 91, is adapted for connection to a source of high voltage (not shown) via a six prong connector 94 located on the plate 42. In addition, the plate 42 has a hole 96 which is of similar size and location to the connector 90 on the plate 40. Connector 90, however, simply fits through the hole 96 and makes no connection with hole 96.

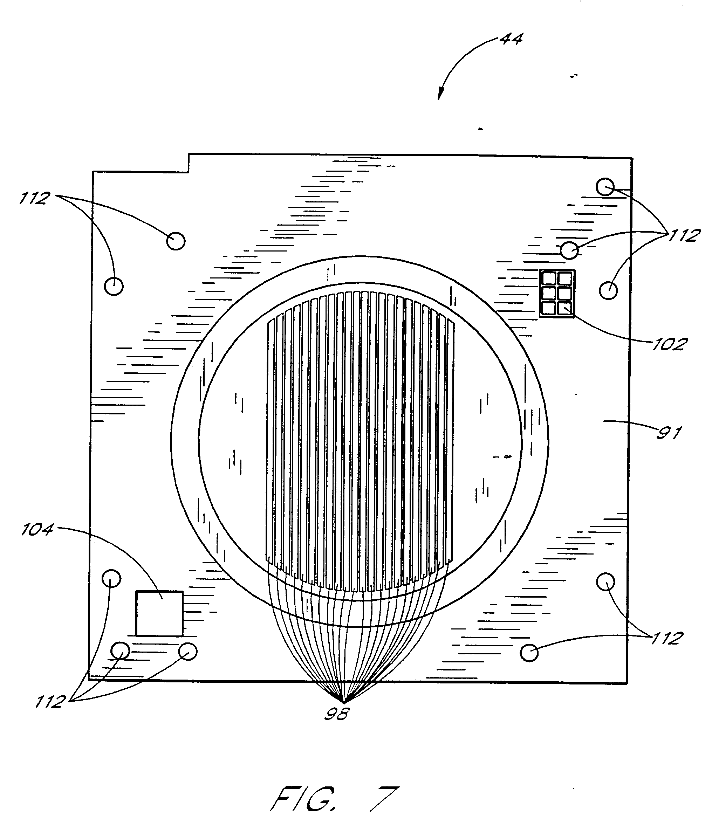

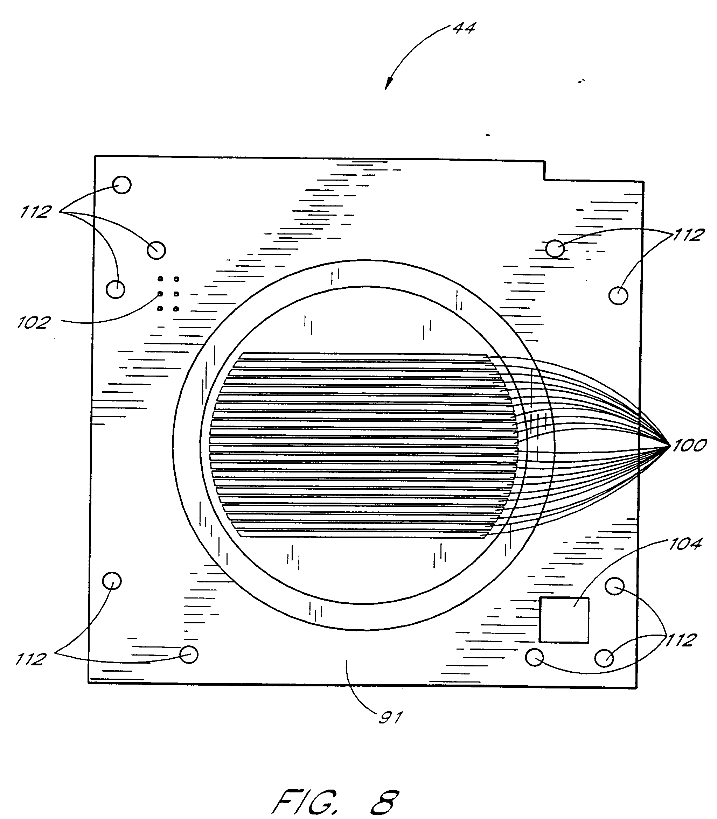

FIGURES 7 and 8 illustrate opposite sides of the plate 44 of the detector assembly 20. FIGURE 7 illustrates the side of the plate 44 facing the second ion chamber 50 and FIGURE 8 illustrates the side of the plate 44 facing the third ion chamber 52. The conductive material facing the second ion chamber 50, as shown in FIGURE 7, is comprised of a plurality of electrically isolated conductive elongate vertical strips 98. The conductive material facing the third ion chamber 52, as shown in FIGURE 8, is also comprised of a plurality of electrically isolated conductive elongate strips 100. The conductive strips 100 facing the third ion chamber 52 are horizontal, and thus are orthogonal (i.e. disposed at right angles) relative to the conductive strips 98 facing the second ion chamber 50.

Still referring to FIGURES 7 and 8, the conductive material 98,100, which is surrounded by mask 91, of the plate 44 is in electronic communication with a signal monitoring device (not shown) via a socket 102 for a six prong connector. The socket 102 of the plate 44 is adapted for connection to the six prong connector 90 of the plate 40. In addition, the plate 44 has a hole 104 which is of similar size and location to the six-prong connector 94 on the plate 42. However, the connector 94 of the plate 42 simply fits through the hole 104. No connection with connector 94 and hole 104 is made.

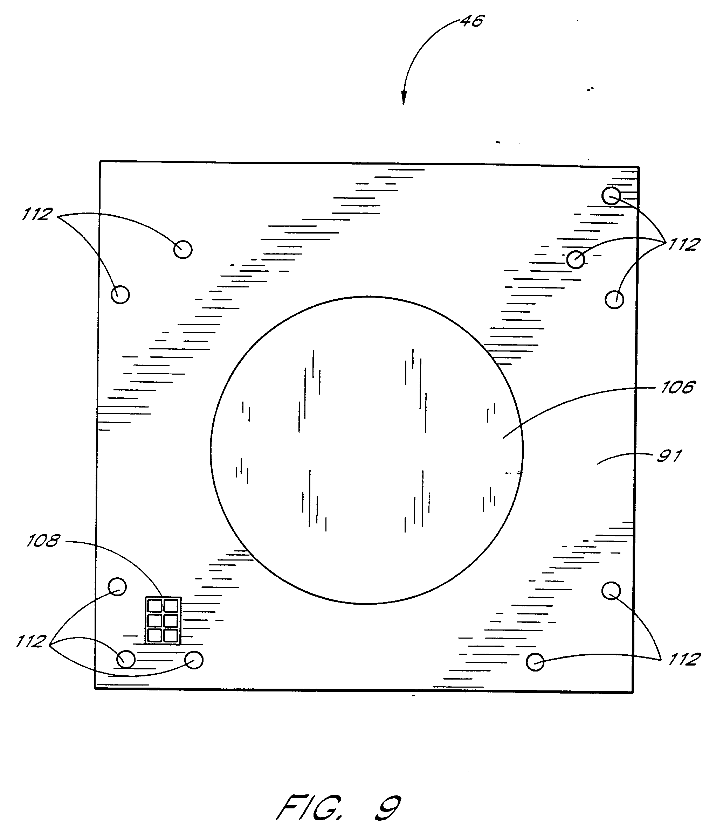

FIGURE 9 illustrates the pattern of conductive material 106 on the plate 46 of the detector assembly 20. The conductive material 106, which is surrounded by mask 91, is located on the front side of the plate 46 and faces the third ion chamber 52. The conductive material pattern, similar to that of the plate 42, is disk-shaped. The conductive material 106 is adapted for connection to a source of high voltage (not shown) via a six hole socket 108 located on the plate 46. The socket 108 is used for connection with the six prong connector 94 of the plate 42. The back side of the plate (not shown) does not have conductive material but is covered with mask 91.

Each of the four plates 40-46 shown in FIGURES 4-9 has reference holes 112 located at the corners of the plates 40-46. The reference holes 112 are used for aligning the plates 40-46 within the detector assembly 20 to the charged particle beam path.

Referring to FIGURES 1-9, during operation of the radiation transport system 10, the accelerator 12 emits a beam of charged particles into the beam transport tube 14. In the embodiment shown in FIGURE 1, only one treatment room 19 will be in use at any one time, and thus, the particles will be transmitted through the tube 14 to only a selected one of the treatment rooms 19. In the selected treatment room, a nozzle receives the beam from the transport tube 14 and directs it to a selected location or treatment site of a patient, for example, the site of a tumor.

Both the accelerator 12 and the beam transport tube 14 are held under vacuum in order to prevent undue scattering or degradation of the charged particle beam. However, small amounts of scattering and beam divergence occur as a result of the scattering characteristics of the charged particles. Beam sensors 18 and magnets 16, located at various intervals along the beam transport tube 14, are used to detect and correct beam shape and position changes. For convenience, these beam sensors 18 and magnets 16 will be referred to using the modifiers first, second, third, etc. depending on their proximity to the accelerator 12. For example, as one moves along the beam path from the accelerator to a selected one of the treatment rooms 19, the first sensor encountered will be termed the "first sensor", the second sensor, the "second sensor", and so forth. Similarly, the detector within the first sensor will be termed the "first detector", the detector within the second sensor will be termed the "second detector", and so on. A similar convention will be used for the magnets 16.

In use, the movable detector assemblies 20 are sequentially lowered in the path of the beam of charged particles, such that no more than one beam detector is in the path of the beam at any one time. Although all the detectors 20 could be placed in the beam path simultaneously, the scatter and beam divergence that occurs after the beam emerges from the detector 20 initially encountered by the beam will seriously affect subsequent results/readings at detectors downstream of the initial detector. Therefore, it is preferred that only one detector at a time be in the beam path and that the "normal" position for the detectors is outside the beam path. In this regard, an example of the preferred process could be to first, move the first detector 20 from a position outside the beam path to a position within the beam path (i.e. to its first position); second, obtain detector readings and compare these readings to desired readings; third, adjust magnets 16 until detector readings are substantially the same as the desired readings; fourth, move the detector 20 out of the beam path (i.e. to its second position); and fifth, repeat the process with the second or subsequent detector.

The anti-vacuum box 32 of the movable detector assembly 20 is actuated to change positions in response to signals sent from the electronic control board 26 to the pneumatic actuator 22. The pneumatic actuator 22 responds to the signals sent from the electronic control board 26 by compressing itself to a final compact state. A bracket 21, attached to and positioned between the pneumatic plunger and the metallic bellows 30, concurrently pushes the detector assembly 20, and subsequently the anti-vacuum box 32, into the beam path as the actuator 22 compresses.

The charged particle beam impinges upon the thin sheet of titanium 39 located within the circular port 38 of the cover plate 36 of the anti-vacuum box 32. The titanium 39, due to its low-density, allows the beam to enter the anti-vacuum box 32 without adversely distorting or scattering the charged particle beam. Housed within the anti-vacuum box 32 are the ionization chambers 48-52 which are used to detect the beam shape and location. Each ionization chamber contains an ionizable substance, such as air, and detector plates 40-46.

After entering the anti-vacuum box 32, the charged particle beam enters the first ion chamber 48. The first ion chamber 48 is comprised of the quad foil or plate 40 and the high voltage plate 42. As the charged particles collide with the air molecules, positive and negative pairs of ions are generated. The high voltage plate 42 generates an electric field which attracts the negative ions and the remaining positive ions are collected on the quad foil 40.

The quad foil 40, which is divided into four wedges 54-60 of conductive material, is used to collect beam centering information. The signals generated by the positive ions impinging on the wedges 54-60 are transferred to a computer. The sums and differences of the charge collected on each of the wedges 54-60 are used to determine the position of the beam.

An example of determining beam centering information is as follows. First, the difference between the sum of the signals collected on the two upper wedges (54,56) and the sum of the signals collected on the two lower wedges (58,60) is calculated. Next, the difference between the sum of the signals collected on the two left wedges (54,60) and the sum of the signals collected on the two right wedges (56,58) is calculated. If the two differences both equal zero, then the charged particle beam is centered and no adjustments have to be made. However, if the differences do not equal zero, then the beam is off-center. Subsequently, the results are analyzed by a computer and appropriate adjustments are made to the magnets 18 to center the beam.

Next, the charged particle beam passes through the conductive material 92 of the plate 42 and continues into the second and third ion chambers 50,52 respectively. The second ion chamber 50 is bounded by the plate 42 and the plate 44, whereas the third ion chamber 52 is bounded by the plate 44 and plate 46. The plate 46, similar to the plate 42, generates an electric field which attracts the negative ions. The plate 44, similar to the quad foil 40, collects the positive ions generated by the beam of charged particles colliding with the molecules of the ionizable substance.

The patterns of conductive material 98,100 on opposite sides of the plate 44 are used to determine the shape of the charged particle beam. For the preferred embodiment of the invention, the conductive material is patterned into strips 100, which are angled relative to one another (e.g. at right angles) on opposite sides of the plate. The amount of charge collected on each strip 100 is analyzed and compared to the desired results. If the amount of charge collected on each strip 100 is similar to the desired amount, then the beam shape is acceptable and no adjustments are made. If, however, the amounts differ, then the beam shape is altered by adjusting the magnets 16 until the two amounts are the same.

After the beam has been analyzed by the beam sensor 18 and the appropriate adjustments have been made via the magnets 16, the movable detector assembly 20 is removed from the path of the charged particle beam. Once again, signals are sent from the electronic control board 26 to the pneumatic actuator 22. The pneumatic actuator 22 responds to the signals sent from the electronic control board 26 by expanding itself to a final extended state. Thus, the bracket 21 concurrently pushes the detector assembly 20, and subsequently the anti-vacuum box 32, out of the beam path as the actuator 22 expands. Once the detector assembly 20 is in its second position, the beam can pass though the cavity previously occupied by the box 32. This cavity, similar to the interior of the beam transport tube 14, is under vacuum.

The beam of charged particles is detected and analyzed at the next beam sensor 18 and the above described detection is repeated. The detection process occurs at each beam sensor 18 location between the accelerator 12 and the selected target site 19. In this regard, it will be understood that all of the sensors 18 and detectors 20 of the preferred embodiment are the same. The beam sensors 18 detect both the shape and position of the charged particle beam. When necessary, adjustments are made to correct errors in beam shape and position, by driving appropriate ones of the magnets 16 so as to reshape or realign the beam.

It will be apparent that numerous variations and modifications can be made without departing from the spirit of the present invention. Therefore, it should be understood that the embodiments of the present invention described above and shown in the figures of the accompanying drawings are illustrative only and are not intended to limit the scope of the present invention.

## Claims

1. A radiation therapy transport apparatus for guiding a beam of charged particles from a charged particle source to a patient at a treatment site, comprising:
a tube connected to receive the charged particles for propagation therein along a beam path;
plural magnets for adjusting parameters of the beam as it propagates within the tube;
a beam sensor mounted on said tube, said sensor comprising a detector, said detector mounted in said sensor such that said detector is movable between a first position and a second position, said first position being in said beam path and said second position being outside said beam path, said detector comprising:
(a) at least one ion chamber which contains a substance ionizable by the charged particle beam;
(b) plural plates which form at least one ion chamber, each of said plates being rigid and including a pattern of conductive material on at least one side thereof, one of the patterns of conductive material comprising a first set of electrically isolated conductive elongate strips, and another of the patterns of conductive material comprising a second set of electrically isolated conductive elongate strips, said first set of strips being disposed at an angle relative to said second set of strips, said sets of strips producing signals in response to ionization of said substance by said beam.

2. The apparatus of Claim 1, wherein said signals vary in amplitude as a function of the shape of the beam such that the amplitude of at least some of said signals changes when the shape of the beam changes.

3. The apparatus of Claim 1, wherein said first and second sets of conductive strips are on opposite sides of a single one of said plural plates.

4. The apparatus of Claim 1, wherein another of the patterns of conductive material comprises at least three electrically isolated conductive portions that produce respective electrical signals in response to ionization of said substance by said beam, each of said signals having a parameter that is dependent on the position of said beam within said tube.

5. The apparatus of Claim 4, wherein said parameter that is dependent on the position of said beam within said tube is amplitude.

6. The apparatus of Claim 4, wherein said electrically isolated portions comprise wedges of said conductive material.

7. The apparatus of Claim 6, wherein each of said wedges has a pair of edges, each of said pair of edges being juxtaposed with an edge of an adjacent wedge.

8. The apparatus of Claim 7, wherein each of said wedges has an apex and wherein the apexes of said wedges are juxtaposed such that said wedges form sections of a disk.

9. The apparatus of Claim 1, wherein a first of said plural plates comprises a pattern of conductive material, and wherein said first and second sets of conductive strips are positioned on opposite sides of a non-conductive substrate, said substrate and said strips forming a second of said plural plates.

10. The apparatus of Claim 9, wherein the first plate is positioned upstream of the incoming beam of charged particles relative to the second plate.

11. The apparatus of Claim 9, wherein the pattern of conductive material on said first plate is comprised of four electrically isolated portions.

12. The apparatus of Claim 11, wherein said electrically isolated portions are separated by gaps, each of said gaps being equal to or less than 0.008 inch.

13. The apparatus of Claim 9, wherein said detector comprises first, second and third ion chambers.

14. The apparatus of Claim 13, wherein said first ion chamber is bounded by said first plate and a third plate.

15. The apparatus of Claim 14, wherein said third plate comprises a disk-shaped pattern of conductive material on each side of a substrate.

16. The apparatus of Claim 15, wherein both of said disk-shaped patterns are adapted for connection to a source of high voltage.

17. The apparatus of Claim 16, wherein said second ion chamber is bounded by the other of said disk-shaped patterns of said third plate and by said second plate.

18. The apparatus of Claim 17, wherein said other of said disk-shaped patterns is positioned upstream of the incoming beam of charged particles relative to said second plate.

19. The apparatus of Claim 18, wherein said third ion chamber is bounded by said second plate and a fourth plate.

20. The apparatus of Claim 19, wherein said fourth plate has a disk-shaped pattern of conductive material which is adapted for connection to a source of high voltage.

21. The apparatus of Claim 19, wherein said second plate of said third ion chamber is positioned upstream of the incoming beam of charged particles relative to said disk-shaped pattern of conductive material of said fourth plate.

22. The apparatus of Claim 21, wherein said plates are separated by about 5mm.

23. The apparatus of Claim 1, wherein said conductive strips and said conductive material are comprised of copper.

24. The apparatus of Claim 1, wherein said conductive strips and said conductive material are comprised of gold.

25. The apparatus of Claim 1, wherein said substance ionizable by the charged particle beam comprises air.
